# EUROPÄISCHE PATENTSCHRIFT

(11) **EP 0 740 522 B1**
(45) Veröffentlichungstag und Bekanntmachung des Hinweises auf die Patenterteilung: **08.03.2000**
(21) Anmeldenummer: 94927510.1
(22) Anmeldetag: 29.08.1994
(51) Int. Cl.: A61B 1/00, H01L 41/09

(54) **Piezoelektrisches Linear-Antriebselement**
Piezo-electric linear drive element
Elément de mouvement linéaire piezo-électrique

(30) Priorität: 30.08.1993 DE 4329162; 30.08.1993 DE 4329163
(43) Veröffentlichungstag der Anmeldung: 06.11.1996
(73) Patentinhaber: STM Medizintechnik Starnberg GmbH, 86947 Schwabhausen/Weil (DE); Gründl und Hoffmann GmbH, 82319 Starnberg (DE)
(72) Erfinder: GRÜNDL, Andreas, 81377 München (DE); BOB, Konstantin, 69469 Weinheim (DE); BOB, Alexander, 68163 Mannheim (DE)
(74) Vertreter: Tiedtke, Harro, Dipl.-Ing.
(86) Internationale Anmeldenummer: EP9402859
(87) Internationale Veröffentlichungsnummer: WO9506428

(56) Entgegenhaltungen:
- EP-A- 0 304 380
- EP-A- 0 362 613
- DE-B- 1 265 302
- US-A- 4 846 573
- US-A- 4 870 951
- US-A- 4 924 852
- US-A- 5 159 446

## Beschreibung

Die Erfindung bezieht sich auf ein miniaturisiertes, piezoelektrisches linear-Antriebselement sowie ein Endoskop mit einem Endoskopschaft, gemäß den Oberbegriffen der Patentansprüche 1 bzw. 7.

Endoskope sind zu einem wichtigen Hilfsmittel in der Technik und in der Medizin geworden, um kanalartige Hohlräume zu inspizieren, die auf andere Weise nicht oder nur mit erheblichen Eingriffen zugänglich sind. Endoskope sind an ihrem Distalende mit einer Beleuchtungseinrichtung und mit einer Optik zur visuellen Erfassung des davor liegenden Bereichs des Hohlraums ausgerüstet. Die am Distalende im vorderen Bereich des Endoskops erfaßte, optische Information wird normalerweise entweder mittels einer Faseroptik durch den Endoskopschaft nach hinten zu seinem Bedienungsende übertragen, oder wird mittels eines Kamerachips am Distalende erfaßt und durch eine elektrische Leitung durch den Endoskopschaft nach hinten geleitet und auf einem Bildschirmmonitor sichtbar gemacht. Insgesamt haben Endoskope, abgesehen von dem hinteren Bedienungsende, üblicherweise eine langgestreckte, biegsam-stabförmige Gestalt.

Ein beweglicher Abschnitt im vorderen Endbereich des Endoskopschafts erweitert die Möglichkeiten bei der Inspektion eines kanalartigen Hohlraums. Bei handelsüblichen Endoskopen kann der bewegliche Abschnitt abgekrümmt werden, insbesondere um einen zu inspizierenden Wandbereich des Hohlraums frontal vor dem Endoskopschaftende zu haben. Handelsübliche Endoskope haben an ihrem hinteren Bedienungsende Drehräder, mittels derer über Bowdenzüge ein Abkrümmen des beweglichen Abschnitts bewerkstelligt werden kann. Das Arbeiten mit den Drehrädern ist ziemlich unpraktisch. Insbesondere bei relativ langen oder bei im Einsatz in Biegungen liegenden Endoskopschäften erschwert die erhebliche Reibung der Bowdenzüge ein feinfühliges und genaues Abkrümmen des beweglichen Abschnitts.

Aus dem Stand der Technik ist daher ein miniaturisiertes, piezoelektrisches Linear-Antriebselement für das Antreiben eines bewegbaren Elements bekannt geworden, wie es beispielsweise in der EP 0362613 als den zum Erfindungsgegenstand nächst kommenden Stand der Technik offenbart ist. Gemäß dieser Druckschrift hat das Linear-Antriebselement axial beabstandete Klemmeinrichtungen, die wechselweise in Eingriff mit dem bewegbaren Element, vorliegend eine Antriebsstange bringbar sind. Die Klemmeinrichtungen, sind in dieser Druckschrift nicht weiter definiert.

Aus der DE-A 12 65 302 ist ebenfalls ein piezoelektrisches Linear-Antriebselement dieser Gattung bekannt, welches gemäß der dort gezeigten Figur 1 prinzipiell den gleichen Aufbau aufweist, wie das Antriebselement gemäß der EP 0365613. In dieser Druckschrift sind indessen die Klemmeinrichtungen als Gleitstützen definiert, welche wiederum als durch Magnetspulen betätigbare elektrische Magnete ausgebildet sind. In anderen Worten ausgedrückt wird eine Kraftübertragung zwischen den Gleitstützen und einem bewegbaren Element auf elektromagnetische Weise bewerkstelligt.

Ausgehend von dem nächst kommenden Stand der Technik ist es eine Aufgabe der vorliegenden Erfindung, das gattungsgemäße piezoelektrische Linear-Antriebselement derart weiter zu entwickeln, daß eine erhöhte Antriebskraft auf ein bewegbares Element übertragbar ist. Es ist eine weitere Aufgabe der Erfindung ein Endoskop zu schaffen, welches ein bequemeres und feinfühligeres Arbeiten ermöglicht.

Diese Aufgabe wird durch das Linear-Antriebselement gemäß dem Patentanspruch 1 sowie einem Endoskop gemäß dem Patentanspruch 7 gelöst.

Die Erfindung besteht demzufolge darin, die Klemmeinrichtungen mit einem Klemmelement zu versehen, das zumindest einen relativ zu dem bewegbaren Element schräg gestellten Klemmeingriffskörper aufweist. Zur Betätigung des Klemmelements bzw. des Klemmeingriffskörpers ist dabei ein Klemmelementbetätiger vorgesehen, mittels dem der Klemmeingriffskörper in Richtung zum bewegbaren Element verschwenkt werden kann, um den Klemmeingriffskörper gegen das bewegbare Element zu pressen. Durch die schwenkbare Anordnung des Klemmeingriffskörpers an dem Klemmelement können große Axialkräfte auf das bewegbare Element über den Klemmeingriffskörper, sowie das Klemmelement vom piezoelektrischen Linearbeweger übertragen werden.

Das erfindungsgemäße Endoskop ist ferner dadurch gekennzeichnet, daß in dem Endoskopschaft mindestens ein miniaturisiertes, elektrisches Antriebselement gemäß der Erfindung zum Bewegen des beweglichen Abschnitts vorgesehen ist.

Das erfindungsgemäße elektrische Bewegen des beweglichen Abschnitts ermöglicht ein sehr viel bequemeres und feinfühligeres Arbeiten. Da die Antriebsbewegungen über kürzere Distanzen zu dem beweglichen Abschnitt übertragen werden, vermindern sich die mit Reibung im Zusammenhang stehenden Probleme. Außerdem sind diejenigen Probleme eliminiert, die mir einer genauen Bewegungsübertragung mittels Bowdenzug über größere Distanz verbunden sind, insbesondere Leerweg durch Spiel des Bowdenzugs in seiner Hülle, Tendenz zur Verkürzung und Begradigung der Bowdenzughülle, und dgl.

Das mindestens eine miniaturisierte, elektrische Antriebselement ist vorzugsweise in der Nähe des beweglichen Abschnitts des Endoskopschafts vorgesehen. Mit dieser Ausdrucksweise soll die Situation umfaßt sein, daß das Antriebselement praktisch unmittelbar hinten anschließend an den beweglichen Abschnitt vorgesehen ist, ja sogar in dem beweglichen Abschnitt selbst untergebracht ist. Es soll aber auch die Situation umfaßt sein, daß das Antriebselement ein Stück nach hinten von dem beweglichen Abschnitt beabstandet ist. Der Abstand sollte allerdings nicht zu groß sein, um den im vorhergehenden Absatz geschilderten Problemen kein zu großes Gewicht zukommen zu lassen.

Es gibt Piezokristalle und -keramiken, die, wenn eine Spannung angelegt wird, die Länge ändern. Zur praktischen Anwendung wird eine Vielzahl dünner Piezokristall- oder Piezokeramikplättchen aufeinandergestapelt und zwischen ihnen jeweils eine Leiterschicht angebracht. Wird nun zwischen jeweils zwei aufeinanderfolgenden Leiterschichten eine Spannung angelegt, so ergibt sich bei passender Orientierung des piezoelektrischen Materials z.B. eine Dehnung des Stapels aus den Piezoplättchen in dessen Längsrichtung. Solche piezoelektrischen Elemente können im Verhältnis zu ihrer Größe sehr große Stellkräfte erzeugen.

Da die Längendehnung beim Anlegen der Spannung nur etwa 1 Promille der Länge des Piezomaterials beträgt, ist es eine günstige Möglichkeit, die nötigen Stellwege mit einer Folge linearer Antriebsschritte zu erzeugen.

Es gibt Fälle, in denen das Antriebselement nur mit Schwierigkeiten dort plazierbar ist, wo die Antriebsbewegung zum Bewegen des beweglichen Abschnitts benötigt wird. Deshalb ist es gemäß Anspruch 7 vorgesehen, zwischen dem Antriebselement und dem beweglichen Abschnitt des Endoskopschafts ein Bewegungsübertragungselement vorzusehen. Das Bewegungsübertragungselemen ist vorzugsweise ein langgestreckter, biegesteifer oder biegeweicher Strang. Ferner kann es bevorzugt sein, als Bewegungsübertragungselement einen flachen Strang beliebiger Breite vorzusehen. Als konkrete Beispiele seien seilartige Zugglieder und Elemente in Form insbeondere dünner Stangen oder Bänder genannt, wobei metallische Materialien, Kunststoffmaterialien, faserverstärkte Materialien und anderes mehr in Frage kommen. Das Antriebselement arbeitet auf das Bewegungsübertragungselement, und dieses ist an den beweglichen Abschnitt des Endoskopschafts angeschlossen.

Vorzugsweise weist das Endoskop eine Positionsbestimmungseinrichtung auf, mit der sich die Position des Bewegungsübertragungselements hinsichtlich seiner Längsbewegung relativ zu dem Antriebselement bestimmen läßt.

Wenn man, wie weiter unten noch genauer ausgeführt wird, in dem Endoskopschaft mehrere Antriebselemente umfangsmäßig beabstandet unterbringt, insbesondere um den beweglichen Abschnitt verkürzen bzw. verlängern und abkrümmen zu können, lassen sich aufgrund der Positionsbestimmung der Bewegungsübertragungselemente die jeweils zugeordneten Antriebselemente derart steuern, daß die Bewegungsübertragungselemente alle identisch vorwärts bewegt oder rückwärts bewegt werden, also der bewegliche Abschnitt des Endoskopschafts gradlinig verlängert oder verkürzt wird. Aufgrund der Positionsbestimmung liegt ferner eine elektrische Information darüber vor, ob der bewegliche Abschnitt tatsächlich gradlinig ausgerichtet ist oder nicht. Im Fall des Abkrümmens des beweglichen Abschnitts ist ein ordnungsgemäßer Funktionsablauf sichergestellt. Ein ziehend arbeitendes Antriebselement liefen nämlich, weil ein Abkrümmwiderstand des beweglichen Abschnitts zu überwinden ist, eine geringere Bewegungsgeschwindigkeit des Bewegungsübertragungselements als ein drückend bzw. herauslassend arbeitendes Antriebselement für die konvexe Seite der Abkrümmung. Für den Abkrümmvorgang lassen sich die Antriebselemente aufgrund der Positionserfassung der Bewegungsübertragungselemente so steuern, daß auf der konkaven Krümmungsseite das Bewegungselement bzw. die Bewegungselemente mit passend korrespondierender Geschwindigkeit nach hinten gezogen werden wie an der konvexen Seite der Abkrümmung das Bewegungsübertragungselement bzw. die Bewegungsübertragungselemente nach vorn geschoben werden. Auch im Fall des Abkrümmens des beweglichen Abschnitts liegt eine elektrische Information über den tatsächlichen Abkrümmungszustand vor, die für den untersuchenden Arzt auf einer Anzeige sichtbar gemacht werden kann. Die angesprochene Steuerung der Antriebselemente auf bestimmte erforderliche Geschwindigkeiten erfolgt vorzugsweise durch Steuern der Frequenz des Bewegungsablaufs des betreffenden Antriebselements.

Als Positionsbestimmungseinrichtung ist vorzugsweise eine induktiv arbeitende Positionsbestimmungseinrichtung vorgesehen. Diese läßt sich insbesondere durch eine Spule verwirklichen, in die das Bewegungsübertragungselement oder ein bestimmter Abschnitt des Bewegungsübertragungselements axial mehr oder weniger tief eintaucht. Durch Messen der momentanen Induktivität der Spule läßt sich die Eintauchlänge des Bewegungsübertragungselements bzw. des Abschnitts des Bewegungsübertragungselements und damit dessen Position hinsichtlich seiner Längsbewegung relativ zu dem Antriebselement bestimmen.

Besonders günstig ist es, wenn die Positionsbestimmungseinrichtung zwei Spulen aufweist, die beidseits des Antriebselements angeordnet sind. Weitere Einzelheiten zu dieser Ausgestaltung werden weiter unten bei den bevorzugten Ausführungsbeispielen ausgeführt.

Das Linear-Antriebselement entwickelt während seines Betriebs Verlustwärme. Insbesondere bei einem piezoelektrischen Antriebselement kann dessen Temperatur aufgrund der Verlustwärme so stark ansteigen, daß sich der für die Funktion erforderliche Piezoeffekt verschlechtert. Deshalb ist es in Weiterbildung der Erfindung bevorzugt, eine Kühlung für das mindestens eine Antriebselement vorzusehen. Die Kühlung läßt sich besonders günstig als Siedebadkühlung ausführen, z.B. mit einer inerten Flüssigkeit, wie einer Fluorkohlenstoff-Flüssigkeit, die bei einer Temperatur, wie beispielsweise etwa 50° C, siedet, die unterhalb der Temperatur der erheblichen Verschlechterung des Piezoeffekts liegt. Die Siedebadkühlung ergibt einen besonders intensiven Wärmeübergang von den piezoelektrischen Bauteilen durch lokales Verdampfen der Kühlflüssigkeit. Die dabei entstehenden Dampfblasen kondensieren in der Kühlflüssigkeit in einiger Entfernung von den eigentlichen Kühlungssteilen. Da die absoluten Verlustleistungen, die durch Kühlung abzuführen sind, recht gering sind, ergibt sich insgesamt keine übermäßige Erwärmung der Kühlflüssigkeit.

Die beschriebene Siedebadkühlung kann man ganz einfach dadurch bewerkstelligen, daß das Innere des Endoskopschafts mindestens im Anordnungsbereich des Antriebselements bzw. der Antriebselemente mit der Kühlflüssigkeit gefüllt wird. Kühlflüssigkeiten der genannten Art haben außerdem hervorragende Isolationseigenschaften, so daß man an Isolationsaufwand für das Antriebselement sparen kann. Schließlich kommt es der engen Einhaltung der Passung für den Klemmvorgang zwischen dem Antriebselement und dem Bewegungsübertragungselement zugute, wenn wegen der Kühlung das Antriebselement in einem relativ engen Temperaturbereich betrieben wird.

Vorzugsweise ist das erfindungsgemäße Linear-Antriebselement mit zwei piezoelektrisch betätigbaren Klemmelementen zum wechselnden Eingriff mit dem Bewegungsübertragungselement auszustatten. Durch Aneinanderreihen von Aktivierungen der beiden Klemmelemente und des piezoelektrischen Linearbewegers wird eine Relativbewegung zwischen Bewegungsübertragungselement und Antriebselement erzeugt. Der Begriff "Relativbewegung" bedeutet, daß sich einerseits das Antriebselement entlang einem stationären Bewegungsübertragungselement bewegen kann, und daß zum anderen das Bewegungsübertragungselement durch ein festgehaltenes Antriebselement verschoben werden kann.

In bevorzugter Ausgestaltung der Erfindung weist das Klemmelement mindestens den einen Klemmeingriffskörper auf, und die piezoelektrische Betätigungsbewegung wird über eine Schrägfläche in die Klemmbewegung des Klemmeingriffskörpers umgesetzt. Je nach Ausmaß der Schrägstellung der Schrägfläche relativ zu der Arbeitsrichtung des piezoelektrischen Klemmelementbetätigers kann man gezielt eine Kraftverstärkung für die Einklemmung des Bewegungsübertragungselements oder eine Wegvergrößerung für die Klemmbewegung des Klemmeingriffkörpers erzeugen. Vorzugsweise sind mehrere Klemmeingriffskörper vorgesehen, die das Bewegungsübertragungselement zwischen sich festklemmen; wenn nur ein Klemmeingriffskörper vorgesehen ist, klemmt dieser das Bewegungsübertragungselement gegen ein Widerlager.

Es ist günstig, wenn sich beim Aufheben der Betätigung des Klemmelements der Klemmeingriff federnd wieder löst. Das Klemmelement kann einstückig oder aus mehreren Teilen zusammengesetzt sein.

In einem idealisierten Klemmelement schwenkt der Klemmeingriffskörper um einen festen Schwenkpunkt. Abhängig von dem Ausmaß der Schrägstellung und den Abständen zwischen Schwenkpunkt und Klemmfläche einerseits und dem Schwenkpunkt und der Stelle, an der die piezoelektrische Betätigungsbewegung am Klemmeingriffskörper eingreift, andererseits kann man durch Hebelwirkung gezielt eine Kraftverstärkung für die Einklemmung des Bewegungsübertragungselements oder eine Wegvergrößerung für die Klemmbewegung erzeugen. Zur Kraftübertragung zwischen dem Klemmelementbetätiger und dem Klemmeingriffskörper kann mindestens eine flexible Verbindung vorgesehen sein. Vorzugsweise ist die Verbindung zwischen Klemmelementbetätiger und Klemmeingriffskörper biegeweich einstöckig vorgesehen. Alternativ kann auch ein einseitig frei anliegendes Tastelement angeordnet sein.

Ferner kann man eine derartige Schrägstellung des Klemmeingriffskörper relativ zu dem Bewegungsübertragungselement vorsehen, daß sich bei einer Kraftübertragung zwischen dem Klemmelement und dem Bewegungsübertragungselement eine Selbstverstärkung des Klemmeingriffs ergibt. Funktionell kommt es hierbei auf den spitzen Winkel zwischen der Längsrichtung des Bewegungsübertragungselements und derjenigen Geraden an, die bei dem Klemmeingriffskörper von dessen Klemmfläche zu dessen virtuellen Schwenkpunkt bei der Klemmbewegung führt, unabhängig von der geometrischen Gestaltung des Klemmeingriffskörpers. Es gibt einen Grenzwinkel, ab dem die Selbstverstärkung einsetzt; die Betätigungskraft des Klemmbetätigers und die aus der Selbstverstärkung herrührende Kraft wirken zur Erzeugung des Klemmeingriffs zusammen. Bei einem noch steileren Winkel wird ein Punkt erreicht, ab dem die Betätigungskraft des Klemmbetätigers nur noch zur Erzeugung eines anfänglichen Klemmeingriffs benötigt wird; die aus der Selbstverstärkung herrührende Kraft kann den weiteren Klemmeingriff allein sicherstellen.

Die vorstehend mehrfach angesprochene Schrägfläche kann an dem Klemmeingriffskörper und/oder einem hiermit zusammenwirkenden Bauteil vorgesehen sein.

Es ist besonders vorteilhaft, die piezoelektrischen Bauteile des piezoelektrischen Antriebselements alle auf einer Seite des Bewegungsübertragungselements anzuordnen. Eine derartige Anordnung hat den Vorteil, daß die Größe des piezoelektrischen Antriebselements, - insbesondere seine Breite bzw. Höhe - deutlich reduziert werden kann. Das erleichtert es wesentlich, mehrere Antriebselemente an gleicher Stelle hinsichtlich seiner Längserstreckung im Endoskopschaft unterzubringen. Zum anderen ergibt sich durch diesen Aufbau eine erhebliche Vereinfachung für die Herstellung der Antriebselemente.

Besonders einfach gestaltet sich der Aufbau des erfindungsgemäßen Antriebselements, wenn der Effekt der Selbstverstärkung nur in einer der beiden Längs-Bewegungsrichtungen des Bewegungsübertragungselements benutzt wird. Bei speziellen Anwendungen, wenn besonders große Kräfte in einer dieser beiden Bewegungsrichtungen erzeugt werden sollen, ist es günstig, beide Klemmelemente "gleichsinnig" anzuordnen.

Am günstigsten ist es, wenn das Klemmelement umfangsmäßig verteilt mehrere Klemmeingriffskörper aufweist. Ein Bewegungsübertragungselement kann dann, mindestens für einen Teil seines Umfangs, von diesen wie von einer Spannzange oder einem Bohrfutter umfaßt und festgeklemmt werden.

Eine als für die praktische Ausführung besonders einfach bevorzugte Ausbildung eines Klemmelements bietet ein, insbesondere in einem Teil seiner Länge, geschlitztes Ringelement.

Die technische Umsetzung ist besonders einfach, wenn das Bewegungsübertragungselement ein langgestreckter Strang ist. Hier sind verschiedenste Lösungen vorstellbar, so kann z.B. ein Zugseil aus Metall oder Kunststoff oder auch eine Stange, die Zug- und/oder Druckkräfte übertragen kann, zum Übertragen der Bewegung verwendet werden.

Einen guten Kompromiß zwischen vielfältiger Bewegungsmöglichkeit und Bauaufwand stellt das Vorsehen von drei Antriebselementen umfangsmäßig beabstandet in dem Endoskopschaft dar; hiermit kann der bewegliche Abschnitt in jede Richtung abgekrümmt werden. Wenn der bewegliche Abschnitt an einer Stelle längenunveränderlich ausgebildet wird, kann man auch mit nur zwei Antriebselementen mit Bewegungsübertragungselementen auskommen; hierbei müssen die Bewegungsübertragungselemente allerdings auch Druckkräfte übertragen können.

Es ist möglich, mehrere der erfindungsgemäßen Antriebselemente nebeneinander vorzusehen, die hinsichtlich der Längserstreckung des Endoskopschafts im wesentlichen an gleicher Stelle angeordnet sind. Alternativ kann man mehrere der Antriebselemente vorsehen, die in Längsrichtung des Endoskopschafts versetzt angeordnet sind. Das ist besonders dann angebracht, wenn bei einer Anordnung an im wesentlichen gleicher Stelle im Endoskopschaft wenig Platz für die Durchführung eines Arbeitskanals verbliebe oder wegen der Größe der Antriebselemente eine Anordnung an im wesentlichen gleicher Stelle des Endoskopschafts nicht möglich ist.

Die beiden wichtigsten, in Ausgestaltung der Erfindung bevorzugten Bewegungsmöglichkeiten des beweglichen Abschnitts sind Abkrümmung und Längenveränderbarkeit. Mit letzterer kann man zur genaueren Inspektion sehr gezielt an einen zu inspizierenden Bereich der Hohlraumwand heranfahren. Beide Bewegungsmöglichkeiten lassen sich auch kombinieren.

Das erfindungsgemäße Antriebselement sowie das hier mit ausgerüstete Endoskop läßt sich zum einen bei der Inspektion, aber auch bei der Durchführung von Arbeiten in technischen Anlagen und Einrichtungen einsetzen. Als Beispiele unter vielen seien Kernreaktoren, chemische Anlagen, Rohrleitungssysteme genannt. Zum anderen läßt sich die Erfindung günstig auf dem Gebiet der Medizin einsetzen, insbesondere zur Exploration von Hohlräumen oder röhrenartigen Kanälen des Körpers oder zur Durchführung von minimal invasiven Operationen. Endoskope haben sich besonders eingeführt zur Exploration der Speiseröhre, des Magens, des Zwölffingerdarms vom Magen aus, des Darms vom Anus aus, der Harnröhre, der Blase und der Harnleiter. Endoskope weisen in der Regel einen sogenannten Arbeitskanal auf, durch den diverse Arbeitselemente eingeführt werden können, z.B. kleine Zangen zur Entnahme von Gewebsproben, Biopsienadeln, beheizbare Schneiddrähte, kleine Scheren, Koagulationselektroden oder dergleichen. Es sei hier darauf hingewiesen, daß auch einige der Arbeitsinstrumente in ähnlicher Art wie der bewegliche Abschnitt des Endoskops durch miniaturisierte, elektrische Antriebselemente betätigt werden können. Schließlich ist in der Regel ein Fluidkanal für Spülflüssigkeit vorhanden, der auch zum Insufflieren mit Luft bzw. speziellen Gasen verwendet werden kann. Insgesamt soll der Begriff "Endoskop" in der vorliegenden Anmeldung auch Geräte umfassen, bei denen der Gesichtspunkt des optischen Inspizierens nicht im Vordergrund steht.

Die Erfindung hat den großen Vorteil, die Reibung entlang der Bewegungsübertragungselemente eines Endoskops speziell beim Einsatz in stark verschlungenen kanalartigen Hohlräumen annähernd auszuschalten, so daß der bewegliche Abschnitt des Endoskops in günstiger Weise genau positioniert werden kann. Der Einsatz eines Endoskops wird damit nicht durch die Länge des zu untersuchenden Hohlraums und dessen Form beeinträchtigt, was besonders bei der der Exploration des menschlichen Enddarms, des Dickdarms und des Dünndarms sowie bei schwierigeren Einsätzen in verschlungenen Hohlraumsystemen wichtig ist.

Die Steuerung der Antriebselemente bzw. des vorderen Abschnitts des Endoskops erfolgt, z.B. mittels eines Steuerknüppels und eine Steuerelektronik präzise und erfordert weniger Aufmerksamkeit. Das bewirkt eine Entlastung des Untersuchenden, der seine Konzentration nicht mehr auf das Positionieren verwenden muß, sondern sich ausschließlich der Untersuchung des Hohlraums zuwenden kann.

Ein weiterer großer Vorteil ist die Längenveränderbarkeit im Bereich des beweglichen Abschnitts, durch die das vordere Ende des Endoskops an einen zu untersuchenden Bereich der Hohlraumwand herangefahren werden kann. Das ist besonders in den Fällen wichtig, wo, wie z.B. bei der Koloskopie, die Wand eine sehr unregelmäßige Struktur aufweist.

Werden hier etwa durch einen Arbeitskanal zusätzliche Arbeitsinstrumente eingeführt, verringert das nahe Heranfahren an den Wandbereich, an dem ein Eingriff vorgenommen werden soll, die Gefahr, einen unbeteiligten Wandbereich mit dem Arbeitsinstrument zu verletzen.

Weiterer Gegenstand der Erfindung ist ein miniaturisiertes, elektrisches Linear-Antriebselement für ein Endoskop mit einem beweglichen Abschnitt im vorderen Endbereich des Endoskopschafts, dadurch gekennzeichnet, daß es als piezoelektrisches Antriebselement einen piezoelektrischen Linearbeweger und zwei im Wechsel zu aktivierende, piezoelektrisch arbeitende Klemmeinrichtungen zur Schaffung von Klemmeingriff mit einem durch das Linear-Antriebselement bewegbaren Gegenstand aufweist.

Es wird ganz besonders betont, daß dieses erfindungsgemäße Linear-Antriebselement nicht nur zum Bewegen des beweglichen Abschnitts eines Endoskopschafts einsetzbar ist, sondern überall dort, wo miniaturisierte, elektrische Linear-Antriebselemente brauchbar sind. Zum Bereich der Erfindung gehören also miniaturisierte, elektrische Liniear-Antriebselemente der im vorstehenden Absatz angegebenen Art für beliebige Einsatzgebiete.

Es wird ferner betont, daß sämtliche spezielleren, für das Antriebselement brauchbaren Ausbildungsmerkmale - selbst wenn sie im Zusammenhang mit dem Endoskop beschrieben sind - auch bei dem Linear-Antriebselement vorgesehen sein können, wenn es für andere Einsatzgebiete als das Endoskop bestimmt ist, und zwar einzeln oder zu mehreren kombiniert.

Bei dem ein Stück weiter vorn angesprochenen Linear-Antriebselement ist allgemein von "einem durch das Linear-Antriebselement bewegbaren Element oder Gegenstand" statt von dem Bewegungsübertragungselement gesprochen worden, weil bei anderen Einsatzgebieten als dem Endoskop häufig kein Bewegungsübertragungselement im engen Wortsinn vorhanden ist.

Die Erfindung und Weiterbildungen der Erfindung werden nachfolgend anhand eines teilweise schematisiert dargestellten Ausführungsbeispiels noch näher erläutert. Es zeigt:
- **Fig. 1**: teilweise geschnitten den vorderen Endbereich eines erfindungsgemäßen Endoskops;
- **Fig. 2**: im Längsschnitt und in vergrößertem Maßstab ein piezoelektrisches Antriebselement, und zwar in der unteren Hälfte eine erste Variante und in der oberen Hälfte eine zweite Variante;
- **Fig. 3**: schematisiert eine Abfolge von Bewegungsschritten, die zu einer Verschiebung des Bewegungsübertragungselements bei einem piezoelektrischen Antriebselement nach **Fig. 2** führt;
- **Fig. 4, 5 und 6**: jeweils im Längsschnitt einen Teil des Eingriffsbereichs zwischen einem Klemmelement und einem Bewegungsübertragungselement;
- **Fig. 7**: in Seitenansicht und teilweise längsgeschnitten ein alternatives Ausführungsbeispiel eines piezoelektrisches Antriebselement;
- **Fig. 8**: einen Querschnitt durch das Antriebselement entlang der Linie 8-8 in **Fig. 7**;
- **Fig. 9**: einen Querschnitt durch das Antriebselement entlang der Linie 9-9 in **Fig. 7**;
- **Fig. 10**: eine schematisierte Darstellung eines Antriebselements mit zugeordneter Positionsbestimmungseinrichtung.

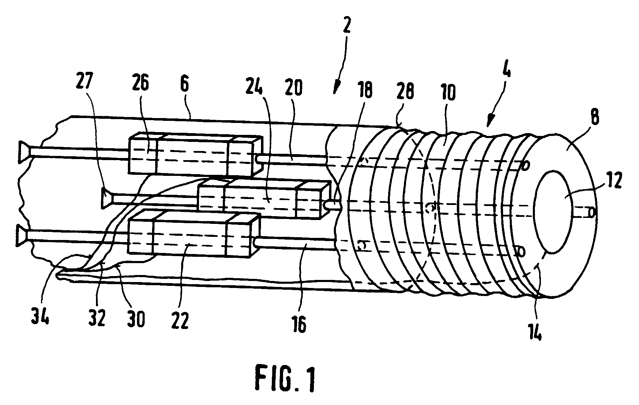

**Fig. 1** zeigt einen Teil eines Endoskopschafts 2 mit einem halbsteifen, biegsamen Hauptabschnitt 6 und einem beweglichen Abschnitt 4 im vorderen Endbereich. Der bewegliche Abschnitt 4 wird im wesentlichen von einem Stützelement gebildet, das mit einem elastischen Material 10, z.B. Gummi, überzogen ist. Das Stützelement kann in der Art einer Schraubenfeder ausgebildet sein. Der bewegliche Abschnitt 4 ist an seinem vorderen Ende 8 verschlossen. Im vorderen Ende 8 ist ein Kamerachip 12 untergebracht, der optische Informationen erfaßt und über eine elektrische Leitung 14 durch den Endoskopschaft nach hinten leitet. Drei Bewegungsübertragungselemente 16, 18, 20 sind an dem vorderen Ende 8 nahe dem Umfang, umfangsmäßig im Winkelabstand von 120° verteilt, befestigt. Die Bewegungsübertragungselemente 16, 18 und 20 sind hinter dem Übergang 28 zwischen dem beweglichen Abschnitt 4 und dem Hauptabschnitt 6 des Endoskopschafts jeweils mit einem miniaturisierten, piezoelektrischen Antriebselement 22, 24, 26 in Eingriff und setzen sich über diese hinaus ein gewisses Stück nach hinten fort. Eine Verdickung 27 ist am hinteren Ende jedes Bewegungsübertragungselements 16, 18, 20 vorgesehen, um zu weites Hindurchbewegen durch die Antriebselemente 22, 24, 26 verhindern.

Die Antriebselemente 22, 24, 26 sind im Hauptabschnitt 6 des Endoskopschafts 2 kurz vor dem Übergang 28 befestigt und erhalten ihre Stromversorgung über elektrische Leitungen 30, 32 und 34. In der gezeigten Ausführungsform sind die Antriebselemente 22, 24, 26 nahe dem Übergang 28 mit im wesentlichen gleichem Abstand von diesem angeordnet, sie können jedoch auch in Längserstreckung gestaffelt angeordnet werden.

Verkürzt man mit einem der Antriebselemente 22, 24, 26 das dazugehörige Bewegungsübertragungselement 16, 18, 20, so führt das zu einem Abkrümmen des beweglichen Abschnitts 4 in derjenigen Axialebene, welche das betreffende Bewegungsübertragungselement enthält. Wenn an mehreren Übertragungselementen gleichzeitig gezogen wird, ergibt sich die Richtung der Abkrümmung als vektorielle Überlagerung. Durch kooperatives Betätigen der Antriebselemente 22, 24, 26 kann der bewegliche Abschnitt 4 somit in jede beliebige Richtung abgekrümmt werden.

Zusätzlich zu dem Kamerachip 12 kann an dem vorderen Ende 8 des Endoskopschafts ein Arbeitskanal enden, in den vom hinteren Ende des Endoskopschafts Arbeitsgeräte eingeführt werden können und der in **Fig. 1** nicht dargestellt ist.

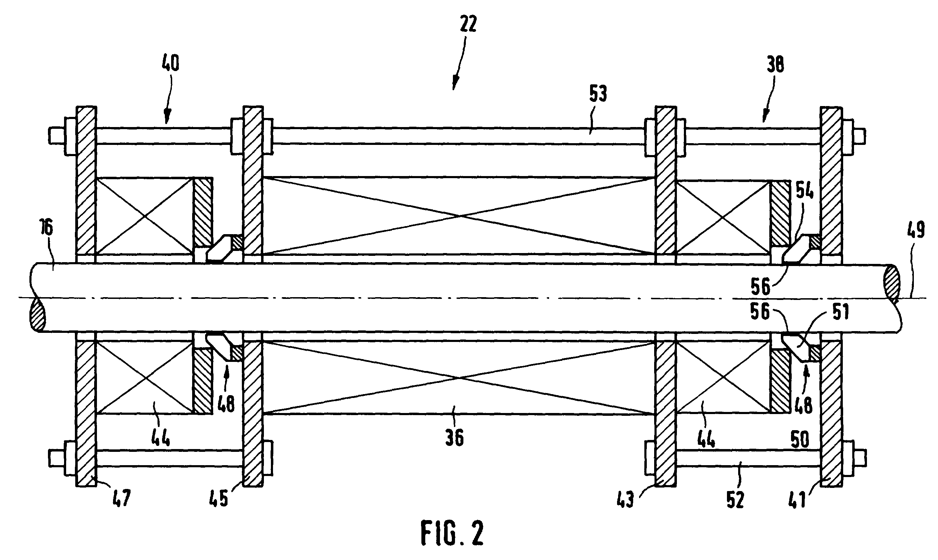

Das in **Fig. 2** gezeichnete Antriebselement 22 besteht im wesentlichen aus einer ersten Klemmeinrichtung 38 (rechts in **Fig. 2**), einer zweiten Klemmeinrichtung 40 (links in **Fig. 2**) und einem dazwischen angeordneten piezoelektrischen Linearbeweger 36. Diese drei Bestandteile sind jeweils, grob gesprochen, hohlzylindrisch und koaxial hintereinander angeordnet. Das Antriebselement 22 weist insgesamt vier Platten auf, nämlich fortschreitend von rechts nach links in **Fig. 2** eine am Ende angeordnete, erste Platte 41, eine zweite Platte 43 zwischen der ersten Klemmeinrichtung 38 und dem Linearbeweger 36, eine dritte Platte 45 zwischen dem Linearbeweger 36 und der zweiten Klemmeinrichtung 40, und am linken Ende eine vierte Platte 47. Die Platten 41, 43, 45, 47 erstrecken sich rechtwinklig zur Längsachse 49 des Antriebselements 22.

Das Bewegungsübertragungselement 16 mit kreisförmigem Querschnitt führt durch einen zentralen Kanal des Antriebselements 22 längs hindurch.

Die Klemmeinrichtungen 38 und 40 sind identisch aufgebaut, so daß es ausreicht, anschließend nur die erste Klemmeinrichtung 38 zu beschreiben.

Die erste Klemmeinrichtung 38 besteht im wesentlichen aus einem piezoelektrischen Klemmelementbetätiger 44 und einem insgesamt ringförmigen Klemmelement 48. Der Klemmelementbetätiger 44 besteht aus einem Stapel piezoelektrischer Scheiben, der sich mit seiner in **Fig. 2** linken Stirnseite gegen die zweite Platte 43 abstützt. Das Klemmelement 48 ist für mehr als die Hälfte seiner axialen Länge durch mehrere, umfangsmäßig verteilte Schlitze geschlitzt, so daß mehrere fingerartige Klemmeingriffskörper 51, jeweils radial innen mit einer Klemmfläche 56 gebildet sind. Das Klemmelement 48 stützt sich mit seiner in **Fig. 2** rechten Stirnseite gegen die erste Platte 41 ab.

Die erste Platte 41 und die zweite Platte 43 sind durch mehrere, um die Längsachse 49 verteilte, axial verlaufende Schrauben 52 miteinander verbunden.

Wenn der Klemmelementbetätiger 44 durch Stromzufuhr aktiviert wird, bewegt sich seine in **Fig. 2** rechte Stirnseite nach rechts; eine dort vorgesetzte Scheibe 50 drückt gegen äußere Schrägflächen 54 aller Klemmeingriffskörper 51 und bewegt deren Klemmflächen 56 im wesentlichen radial nach innen, so daß diese Klemmflächen 56 mit dem Außenumfang des Bewegungsübertragungselements 16 in reibschlüssigen, klemmenden Eingriff kommen. Wenn der Klemmelementbetätiger 44 deaktiviert wird, bewegen sich die Klemmeingriffskörper 51 aufgrund ihrer Eigenelastizität wieder zurück in die radial äußere Ausgangsposition.

Die zweite Klemmeinrichtung 40 ist in gleicher Richtung orientiert wie die erste Klemmeinrichtung 38. Somit stützt sich die stationäre Stirnseite des Klemmelementbetätigers 44 der zweiten Klemmeinrichtung 40 gegen die vierte Platte 47 ab und stützt sich das Klemmelement 48 der zweiten Klemmeinrichtung 40 mit seiner stationären Stirnfläche gegen die dritte Platte 45 ab.

Zwischen der zweiten Platte 43 und der dritten Platte 45 ist der Linearbeweger 36, wiederum ausgebildet als ein Stapel von piezoelektrischen Scheiben, spielfrei angeordneet. Der Linearbeweger 36 ist in Axialrichtung deutlich länger als die Klemmeinrichtungen 38 und 40. Bei der oben in **Fig. 2** dargestellten Variante sind die zweite Platte 43 und die dritte Platte 45 durch mehrere, um die Längsachse 49 verteilte Schrauben 53 miteinander verbunden. Die weiter vorn beschriebenen Schrauben 52 und die Schrauben 53 können einheitliche, über die ganze Länge des Antriebselements 22 durchgehende, gewindestangenartige Bauteile sein. Bei der Variante unten in **Fig. 2** fehlen die Schrauben 53. Bei dieser Variante sind die Stirnseiten des Linearbewegers 36 mit der jeweils zugeordneten Platte 43 bzw. 45 fest verbunden, während bei der oben in **Fig. 2** dargestellten Variante dort keine feste Verbindung erforderlich ist. Wenn Schrauben 53 vorgesehen sind, werden diese in ihrem Durchmesser so bemessen, daß sie sich bei Aktivierung des Linearbewegers 36 dehnen, also eine Auseinanderbewegung der zweiten Platte 43 und der dritten Platte 45 zulassen; bei Deaktivierung des Linearbewegers 36 erfolgt eine elastische Zusammenziehung der Schrauben 53. Im Gegensatz hierzu sind die Schrauben 52 der Klemmeinrichtungen 38, 40 so ausgelegt, daß sie sich nicht erheblich in Axialrichtung dehnen, wenn der Klemmelementbetätiger 44 aktiviert wird; eine gewisse Dehnung der Schrauben 52 ist nicht störend.

Die lineare Betätigungsbewegung eines Klemmelementbetätigers 44 beträgt etwa 1/1000 der Länge des piezoelektrischen Materials im Klemmelementbetätiger 44. Der Abstand, um den sich die Klemmflächen 56 des Klemmelements 48 bei Aktivierung des Klemmelementbetätigers 44 aufeinander zu bewegen, hängt von der axialen Länge des piezoelektrischen Materials im Klemmelementbetätiger 44 und der Neigung der Schrägflächen 54 gegen die Längsachse 49 ab.

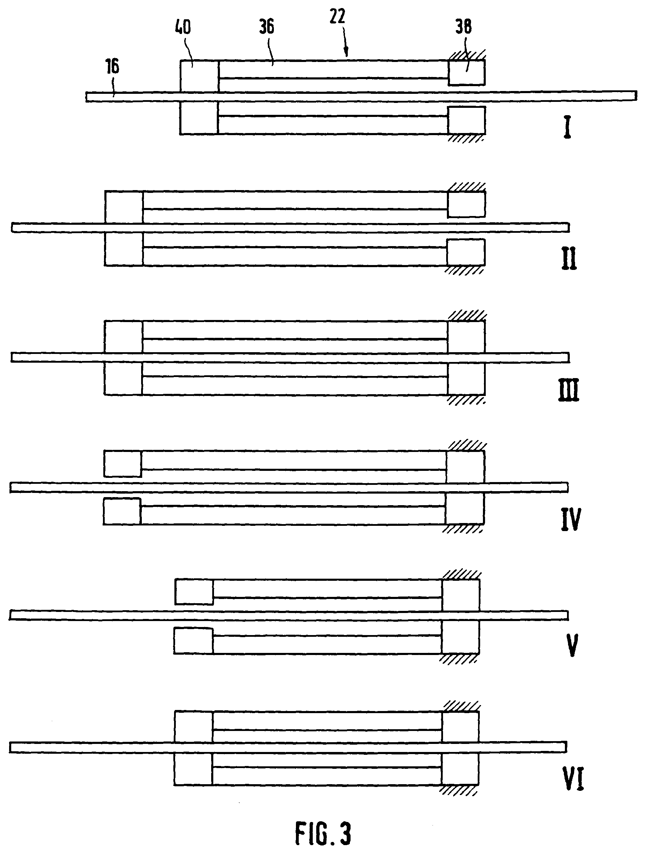

**Fig. 3** zeigt eine Abfolge von Bewegungsschritten I bis VI, die zu einer Bewegung des Bewegungsübertragungselements 16 durch das Antriebselement 22 führt. In I sind an dem schematisiert gezeichneten Antriebselement 22 die Klemmeinrichtungen 38, 40 sowie der piezoelektrische Linearbeweger 36 bezeichnet. Das Antriebselement 22 ist im Bereich der Klemmeinrichtung 38 fest mit dem Hauptabschnitt 6 des Endoskopschafts verbunden, was in **Fig. 1** und **2** nicht eingezeichnet ist, aber in **Fig. 3** durch Schraffur angedeutet ist. Für diese feste Verbindung kann man z. B. an einer oder beiden der Platten 41, 43 ansetzen.

In der Position I befindet sich die Klemmeinrichtung 40 in reibschlüssigem Eingriff mit dem Bewegungsübertragungselement 16, der Linearbeweger 36 ist ohne angelegte Spannung, die Klemmeinrichtung 38 ist nicht betätigt. Wird der Linearbeweger 36 betätigt, so erfährt dieser eine - vergrößert dargestellte - Linearbewegung und verschiebt damit die Klemmeinrichtung 40 relativ zu der fixierten Klemmeinrichtung 38. Mit dieser Verschiebung der Klemmeinrichtung 40 wird auch das Bewegungsübertragungselement 16 nach links gezogen. Dieser Zustand ist in Position II dargestellt. Eine erneute lineare Verschiebung des Bewegungsübertragungselements 16 nach links kann erst durchgeführt werden, wenn der Linearbeweger 36 sich relativ zu dem Bewegungsübertragungselement 16 wieder in seine Ausgangsposition zurückbewegt hat. Dazu ist eine Reihe von Klemmvorgängen nötig, die in den Positionen III und IV gezeigt sind. Die fixierte Klemmeinrichtung 38 wird aktiviert und dadurch mit dem Bewegungsübertragungselement 16 reibschlüssig verbunden. Dann wird die Klemmeinrichtung 40 deaktiviert; der deaktivierte Linearbeweger 36 geht in seine ursprüngliche verkürzte Position zurück. Aus der nun erreichten Position V sind wieder zwei Umklemmschritte der Klemmeinrichtungen 40 und 38 nötig, nämlich Schließen der Klemmeinrichtung 40 und Öffnen der Klemmeinrichtung 38, um in die Ausgangsposition I zu kommen, woraufhin in einer erneuten Schrittfolge eine weitere Verschiebung des Bewegungsübertragungselements 16 vorgenommen wird.

Die Stellvorgänge der beteiligten Piezoelemente 36, 44 erfolgen sehr schnell, so daß eine Wiederholung dieser Schrittfolge eine ausreichende Stellgeschwindigkeit für das Bewegungsübertragungselement 16 ergibt.

Erfolgt die Abfolge der Bewegungsschritte I bis VI in umgekehrter Reihenfolge, wird das Bewegungsübertragungselement 16 von links nach rechts statt wie beschrieben von rechts nach links bewegt. Um diese Bewegung in eine Bewegung des Endes 8 des beweglichen Abschnitts 4 umzusetzen, muß entweder das Bewegungsübertragungselement zur Übertragung von Druckkräften geeignet ausgebildet sein oder muß eine federnde Auseinanderbewegung des Stützelements 10 die notwendige Bewegungskraft liefern, wobei das Bewegungsübertragungselement als eine Art gesteuerte Bremse wirkt.

Wie im vorhergehenden bereits beschrieben, führt das Verkürzen eines der Bewegungsübertragungselemente 16, 18, 20 durch sein dazugehöriges Antriebselement 22, 24, 26 zu einem Abkrümmen des beweglichen Abschnitts 4 gegenüber dem Hauptabschnitt 6 des Endoskopschafts 2. Werden alle drei Bewegungsübertragungselemente 16, 18, 20 um die gleiche Länge gekürzt, so führt dies zu einer linearen Verkürzung des beweglichen Abschnitts 4. Erfolgt diese Verkürzung des beweglichen Abschnitts 4 des Endoskopschafts 2 gegen die Federkraft des Stützelements des beweglichen Abschnitts 4, kann durch ein Nachlassen der Bewegungsübertragungselemente 16, 18, 20 der bewegliche Abschnitt 4 teleskopartig nach vorne verfahren werden. Die Kombination aus Abkrümmbewegung und Längenveränderung ermöglicht eine genaue Positionierung des vorderen Endes 8 des beweglichen Abschnitts 4 des Endoskopschafts 2 an einen zu untersuchenden Wandbereich des Hohlraums.

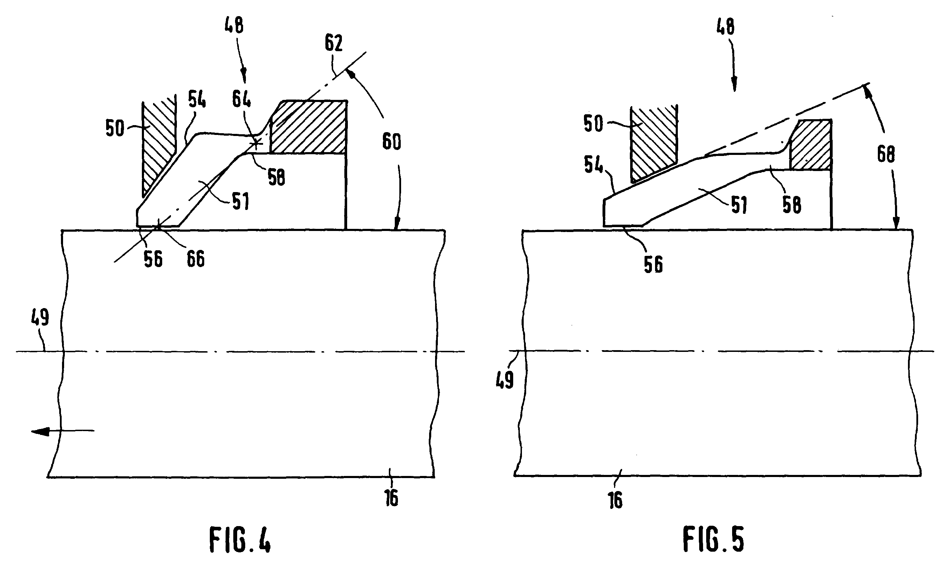

In **Fig. 4** ist nochmals in größerem Maßstab die Geometrie des Klemmelements 48 aus **Fig. 2** und dessen Eingriffsbereich mit dem Ring 50 dargestellt. Ehe die fingerartigen Klemmeingriffskörper 51 in den ungeschlitzten Bereich des ringartigen Klemmelements 48 übergehen, weisen sie einen Bereich 58 vergleichsweise geringer radialer Dicke auf. In dem Bereich 58 findet im wesentlichen die elastische Verformung der Klemmeingriffskörper 51 beim Schließen und Öffnen statt. Eine Gerade ist durch einen repräsentativen Zentralpunkt 64 des Bereichs 58 und einen repräsentativen Zentralpunkt 66 der Klemmfläche 56 gelegt. Die Gerade 62 bildet mit der Längsachse 49 bzw. der Umfangsfläche des Bewegungsübertragungselements 16 einen Winkel 60, der so groß ist, daß sich eine Selbstverstärkung des Klemmeingriffs ergibt. Diese Selbstverstärkung ist nur in einer Axialrichtung wirksam, nämlich bei Verschiebung des Bewegungsübertragungselements 16 nach rechts in **Fig. 4** durch Axialverschiebung der Klemmeinrichtung 48 nach links in **Fig. 4**, oder - in anderer Betrachtungsweise - bei Zug an dem Bewegungsübertragungselement 16 durch eine äußere Kraft in Richtung nach rechts in **Fig. 4**. Mittels des Klemmelementbetätigers 44 muß über den Ring 50 und die Schrägfläche 54 nur noch so viel radiale Anpreßkraft auf die umfangsmäßig verteilten Klemmeingriffskörper 51 aufgebracht werden, daß zwischen den Klemmflächen 56 und dem Außenumfang des Bewegungsübertragungselements 16 eine anfängliche Reibungskraft aufgebaut wird. Wenn dann das Klemmelement 48 in Axialrichtung nach links in **Fig. 4** verschoben wird, verstärkt sich die auf die Klemmflächen 56 wirkende Anpreßkraft von selbst, so daß im Prinzip Kräfte in Axialrichtung von beliebiger Größe zwischen dem Klemmelement 48 und dem Bewegungsübertragungselement 16 übertragen werden können.

Wenn der beschriebene Selbstverstärkungseffekt bei einer bestimmten Anwendung nicht von erheblicher Bedeutung ist, kann man die beiden Klemmeinrichtungen 38 und 40 spiegelbildlich zueinander ausbilden statt gleichsinnig, wie es in **Fig. 2** gezeichnet ist.

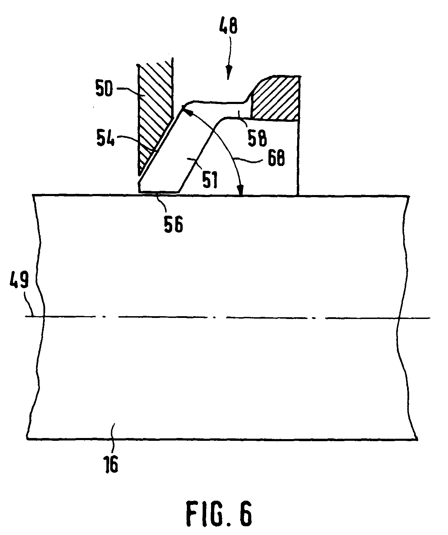

In den **Fig. 5** und **6** ist veranschaulicht, daß man es durch die Wahl des Winkels 68 zwischen der Schrägfläche 54 der Klemmeingriffskörper 51 des Klemmelements 48 und der Längsachse 49 bzw. der Umfangsfläche des Bewegungsübertragungselements 16 in der Hand hat, ob man lieber eine Vergrößerung des im wesentlichen radial verlaufenden Anpreßwegs der Klemmfläche 56 oder eine Vergrößerung der radialen Anpreßkraft der Klemmfläche 56 hat. Wenn der Winkel 68 45° beträgt, entsprechen sowohl der Anpreßweg als auch die Anpreßkraft der Klemmfläche 56 dem axialen Weg und der in Axialrichtung aufgebrachten Kraft von dem Klemmelementbetätiger 44. Wenn hingegen der Winkel 68 kleiner als 45° ist (vgl. **Fig. 5**), erreicht man eine Erhöhung der Anpreßkraft verglichen mit der von dem Klemmelementbetätiger 44 gelieferten, in Axialrichtung wirkenden Kraft, selbstverständlich um den Preis einer Verringerung des Weges der Klemmfläche 56 im Vergleich zu dem Weg des Rings 50 in Axialrichtung.

Wenn hingegen der Winkel 68 größer als 45° ist (vgl. **Fig. 6**) erreicht man eine Vergrößerung des bei Aktivierung des Klemmelementbetätigers 44 von den Klemmflächen 56 im wesentlichen in Radialrichtung zurückgelegten Weges im Vergleich zum Weg des Rings 50 in Axialrichtung. Im Gegenzug nimmt aber die radiale Anpreßkraft im Vergleich zu der vom Klemmelementbetätiger 44 in Axialarichtung gelieferten Kraft ab.

Bei den **Fig. 5** und **6** wurde wiederum zeichnerisch veranschaulicht, daß sich die fingerartigen Klemmeingriffskörper 51 beim Klemmen und beim Lösen schwenkend im wesentlichen um den repräsentativen Punkt 64 bewegen.

Bei den **Fig. 4, 5, 6** ist die Bohrung des Rings 50 jeweils so gezeichnet, daß deren Wand entsprechend dem Schrägungswinkel der Schrägfläche 54 schräggestellt ist. Dies ist keine zwingende Ausbildung. Bedingung ist lediglich, daß die Bohrung des Rings in technisch sinnvoller Weise mit der Schrägfläche 54 in Eingriff ist. Auch die Schrägfläche 54 muß nicht zwingend kegelförmig verlaufen. Z. B. wäre ein äquivalenter, konvex-bogenförmiger Verlauf möglich. Schließlich kann man die Verhältnisse sozusagen umdrehen, also die funktionserforderliche Schrägfläche an dem Ring 50 vorsehen und das, z.B. nur für Linienkontakt vorgesehene, Gegenstück an den Klemmeingriffskörpern 51.

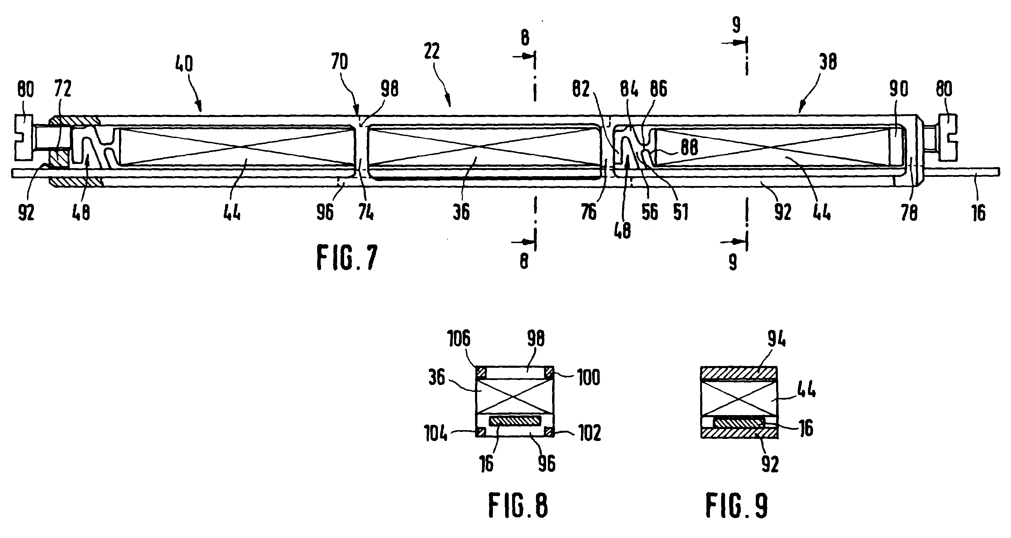

In den **Fig. 7** bis **9** ist ein alternatives Ausführungsbeispiel eines piezoelektrisches Antriebselement dargestellt. Ähnliche Teile sind mit den gleichen Bezugszeichen wie in den **Fig. 1** bis **6** versehen.

Das Antriebselement 22 enthält eine erste Klemmeinrichtung 38, eine zweite Klemmeinrichtung 40 und dazwischen angeordnet den piezoelektrischen Linearbeweger 36. Die piezoelektrischen Klemmelementbetätiger 44 der ersten und der zweiten Klemmeinrichtung 38, 40 sowie der Linearbeweger 36 sind in **Fig. 7** über dem Bewegungsübertragungselement 16 angebracht. Das Antriebselement 22 besitzt ein Gehäuse 70, z.B. aus Metall, das - analog zu den Platten 41, 43, 45, 47 der **Fig. 2** - von links nach rechts Stützwände 72, 74, 76, 78 aufweist. Zwischen den Stützwänden 74 und 76 ist der Linearbeweger 36 spielfrei gehalten. Die Stützwände 76 und 78 bzw. 72 und 74 dienen jeweils zum Abstützen von Bauteilen der ersten bzw. zweiten Klemmeinrichtung 38, 40. In dem unteren Bereich des Gehäuses 70 weisen die Stützwände 72, 74, 76 und 78 jeweils eine rechteckige Öffnung 92 auf, durch die das Bewegungsübertragungselement 16 geführt ist.

Das Klemmelement 48 - funktional entsprechend dem ringartigen Klemmelement 48 der Fig. 2 bis 6 - der Klemmeinrichtung 38 hat eine Basis 82, die mit ihrer in **Fig. 7** linken Stirnseite an der Stützwand 76 anliegt und an ihrem oberen Ende mit einem fingerartigen Klemmeingriffskörper 51 federnd elastisch verbunden ist. Der Klemmeingriffskörper 51 stützt sich mit einer Nase 84 nach oben gegen das Gehäuse 70 ab und trägt an seinem freien Ende die Klemmfläche 56. Insgesamt ragt der Klemmeingriffskörper 51 von oben schräg nach unten gegen das Bewegungsübertragungselement 16. Durch eine biegeweiche Verbindung 86 und ein Anschlußstück 88 ist das Klemmelement 48 mit dem linken Ende des Klemmelementbetätigers 44 fest verbunden. Der Klemmelementbetätiger 44 wird durch eine Justierschraube 80, die mit einem Gewindebereich der rechten Stützwand 78 zusammenwirkt, gestützt. Ein Zwischenstück 90 schützt den Klemmelementbetätiger 44 vor Beschädigungen durch die Justierschraube 80.

Wird der Klemmelementbetätiger 44 betätigt, drückt er mit seinem linken Ende über die biegeweiche Verbindung 86 die Klemmfläche 56 des Klemmeingriffskörpers 51 gegen das Bewegungsübertragungselement 16, dabei verbinden die Nase 84 ein Ausweichen nach oben. Die Grundplatte 92 des Gehäuses 70 erfüllt die Funktion eines Widerlagers für das Bewegungsübertragungselement 16.

Ist die biegeweiche Verbindung 86, welche die Kraft des Klemmelementbetätigers 44 überträgt, weiter nach oben im Verhältnis zur Darstellung in **Fig. 7** versetzt, vergrößert sich bei gegebenem Hub des Klemmelementbetätigers 44 der Anpreßweg, um den sich die Klemmfläche 56 auf das Bewegungsübertragungselement 16 zubewegt, gleichzeitig verringert sich die dabei übertragene Anpreßkraft. Ähnlich den Ausführungen zu **Fig. 5** und **6** kann - hier durch geeignete Wahl der Schrägstellung und der Hebelverhältnisse - eine erwünschte Anpreßkraft-Anpreßweg-Relation gewählt werden.

Wie in **Fig. 2** sind die Klemmeinrichtungen 38 und 40 gleichsinnig orientiert. Die Justierschraube 80 wirkt in der zweiten Klemmeinrichtung 40 mit dem Klemmelement 48 zusammen. In dem Bereich der Stützwand 72 ist das Antriebselement 22 im Längsschnitt dargestellt, so daß die Öffnung 92 in der Stützwand 72 sichtbar ist, durch die das Bewegungsübertragungselement 16 führt.

Im Querschnitt der **Fig. 9** erstreckt sich der Klemmelementbetätiger 44 über die gesamte Breite des Gehäuses 70, so daß das Antriebselement 22 nach unten von der Grundplatte 92 und nach oben von der Deckplatte 94 durch das Gehäuse 70 begrenzt ist. Zwischen Grundplatte 92 und Klemmelementbetätiger 44 ist das Bewegungsübertragungselement 16 vorgesehen, das in der Form eines flachen Bandes mit rechteckigem Querschnitt ausgebildet ist. Die Breite des flachen Bandes ist frei wählbar. In einem Extremfall wirkt nur ein Randbereich des Bewegungsübertragungselements 16 mit dem Antriebselement 22 zusammen. Das Antriebselement 22 ist dann einseitig durchgehend geschlitzt.

Einen Querschnitt durch das Antriebselement 22 im Bereich des piezoelektrischen Linearbewegers 36 zeigt die **Fig. 8**. In diesem Bereich bilden Eckstäbe 100, 102, 104 und 106 eine dehnbare, elastische Verbindung zwischen den beiden Klemmeinrichtungen 38, 40. Der obere Durchbruch 98 und der untere Durchbruch 96 sind in **Fig. 7** durch gestrichelte Linien angedeutet.

Die Funktion dieses Antriebselements 22 erfolgt in Analogie zu der des in Verbindung mit den **Fig. 2** bis **6** beschriebenen Antriebselements 22.

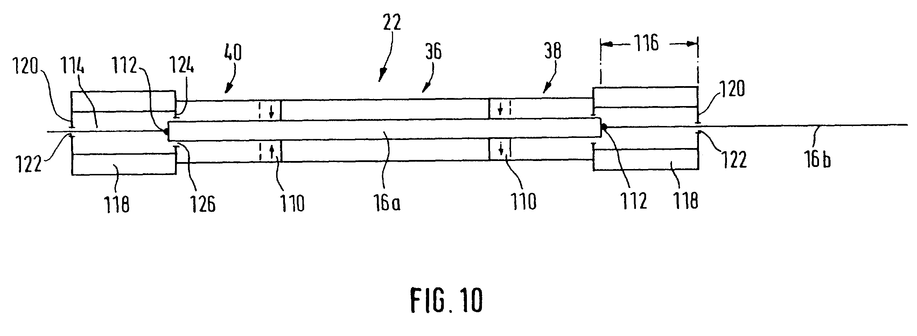

Das Ausführungsbeispiel gemäß **Fig. 10** dient in erster Linie der Erläuterung einer bevorzugten Positionsbestimmungseinrichtung. Teile, die funktional Teilen der vorhergehenden Ausführungsbeispiele entsprechen, sind mit den gleichen Bezugszeichen wie vorher versehen.

Das schematisch dargestellte Antriebselement 22 enthält, wie bei den vorherigen Ausführungsbeispielen, die erste Klemmeinrichtung 38, die zweite Klemmeinrichtung 40 und dazwischen angeordnet den piezoelektrischen Linearbeweger 36. Allerdings sind die durch die Pfeile 110 angedeuteten Klemmelemente jetzt gegensinnig orientiert und beide anschließend an den Linearbeweger 36 angeordnet.

Das Bewegungsübertragungselement 16 besteht bei diesem Ausführungsbeispiel in demjenigen Abschnitt seiner Länge, der mit dem Antriebselement 22 unmittelbar zusammenwirkt, aus einem flachen Band 16a (wie beim Ausführungsbeispiel gemäß **Fig. 7-9** erläutert) und im übrigen aus einem bei 112 an das Band 16a angelöteten Stahlseil 16b, welches zu dem vorderen Ende 8 des beweglichen Abschnitts 4 des Endoskopschafts 2 führt. Am entgegengesetzten Ende des Bandes 16a ist ein Stück Führungsdraht 114 angelötet. In **Fig. 10** ist die Mittelstellung des Bandes 16a gezeichnet, aus der es mittels des Antriebselements 22 um eine Bewegungsstrecke 116 sowohl nach links als auch nach rechts bewegbar ist.

Sowohl am rechten bzw. vorderen Ende als auch am linken bzw. hinteren Ende des Antriebselements 22 ist jeweils eine Spule 118 angesetzt. Im Betrieb werden die beiden Spulen 118 von Wechselstrom durchflossen, und ihr Wechselstromwiderstand wird jeweils in einer Auswerteschaltung bestimmt, wobei der gemessene Wechselstromwiderstand davon abhängt, wie weit das Band 16a in die betreffende Spule 118 in Axialrichtung hineinragt. Bei der gezeichneten Mittelstellung des Bandes 16a ragen dessen beiden Enden nur geringfügig in die beiden Spulen 118. Wird jetzt das Band 16a z.B. nach rechts bewegt, ändert sich die Induktivität der rechten Spule 118. Durch die beschriebene Widerstandsmessung der rechten Spule 118 läßt sich mit guter Genauigkeit bestimmen, um welchen Weg sich das Band 16a aus der Mittelstellung bewegt hat. In analoger Weise kann das Band 16a aus der gezeichneten Mittelstellung mittels des Antriebselements 22 nach links bewegt werden, wobei das Band 16a mit zunehmender Länge in die linke Spule 118 eintritt und durch Widerstandsmessung an der linken Spule 118 die Position des Bandes 16a bestimmt werden kann.

Die beiden Spule 118 weisen jeweils an ihrem dem Antriebselement 22 abgewandten Ende eine Abschlußwand 120 mit einer zentralen, kleinen, runden Öffnung 122 auf. Das Stahlseil 16b bzw. der Führungsdraht 114 können die jeweilige Öffnung 122 passieren, das Band 16a jedoch nicht, wodurch mechanische Endanschläge für die Bewegung des Bandes 16a und damit des gesamten Bewegungsübertragungselements 16 gebildet sind. Die Spulen 118 weisen auch an dem dem Antriebselement 22 zugewandten Ende jeweils eine Abschlußwand 124 mit einer im zentralen Bereich vorgesehenen Durchgangsöffnung 126 auf. Die Durchgangsöffnungen 126 sind allerdings so groß, daß sie den Durchtritt des Bandes 16a erlauben. Der Führungsdraht 114, der seinerseits in der Öffnung 122 und/oder der Öffnung 126 geführt ist, hat die Aufgabe, das linke Ende des Bandes 16a bei dessen Bewegung nach links ordnungsgemäß wieder in die linke Spule 118 hineinzuleiten.

Wenn die beiden Spulen 118 gleiche Induktivität bzw. gleichen gemessenen Widerstand haben, liegt also ein Ermittlungssignal der Positionsbestimmungseinrichtung vor, daß sich das Bewegungsübertragungselement in seiner Mittelstellung befindet. Wenn für alle drei Antriebselemente 22, 24, 26 (siehe **Fig. 1**) dieses Signal vorliegt, befindet sich der bewegliche Abschnitt 4 in seiner Geradeausstellung. An die Auswerteschaltung der Spulen 118 kann eine Wegbegrenzungsschaltung angeschlossen sein, welche die Stromzufuhr zu dem Antriebselement 22 unterbricht, sobald das Band 16a die linke oder die rechte Endstellung erreicht hat, damit das Band 16a nicht aus den Klemmbereichen der Klemmeinrichtungen 38 und 40 herausläuft.

An die Auswerteschaltung der Spulen 118 kann ferner eine Bewegungsüberprüfungsschaltung angeschlossen sein. Wenn das Band 16a trotz Stromzufuhr zu dem Antriebselement 22 keinen adäquaten Weg macht, liegt eine Blockierung des Bewegungsübertragungselements 16 gegen Durchführung der Bewegung vor. Ein fortgesetzter Betrieb des Antriebselements 22 würde zur Ausbidlung von Bremsmarken auf dem Band 16a in den Bereichen des Eingriffs mit den Klemmeinrichtungen 38 und 40 führen. In einer derartigen Situation kann die Bewegungsüberprüfungsschaltung die Stromzufuhr zu dem Antriebselement 22 unterbrechen. Anschließend kann das Band 16a ein kleines Stück in die entgegengesetzte Richtung bewegt werden; danach kann ein Neustart der Bewegung in die vorher beabsichtigte Richtung erfolgen.

Einstellmittel bzw. Nachsteilmittel für die Ruheposition der Klemmeinrichtungen 38 und 40 und damit für das Spiel zwischen den Klemmflächen 56 und dem Bewegungsübertragungselement 16 im ungeklemmten Zustand sowie für die Anpreßkraft zwischen den Klemmflächen 56 und dem Bewegungsübertragungselement 16 im geklemmten Zustand sind ein bevorzugtes Merkmal des erfindungsgemäßen Antriebselements 22. Ein Beispiel hierfür sind die in **Fig. 7** gezeichneten Justierschrauben 80.

## Patentansprüche

1. Miniaturisiertes, piezoelektrisches Linear-Antriebselement (22) für das Antreiben eines bewegbaren Elements (16) beispielsweise zur Bewegungsübertragung auf einen Endoskopschaft mit
einer ersten und einer zweiten mit dem bewegbaren Element (16) wechselweise in Eingriff bringbaren Klemmeinrichtung (38, 40) und einem zwischen den Klemmeinrichtungen (38, 40) angeordneten piezoelektrischen Linearbeweger (36)
**dadurch gekennzeichnet, daß**
jede Klemmeinrichtung (38, 40) ein durch einen piezoelektrischen Klemmelementbetätiger (44) aktivierbares Klemmelement (48) hat, das zumindest einen relativ zum bewegbaren Element (16) schräg gestellten Klemmeingriffskörper (51) aufweist, der unter der Wirkung des Klemmelementbetätigers (44) in Richtung zum bewegbaren Element (16) verschwenkbar ist, um mit dem bewegbaren Element (16) in Klemmeingriff zu kommen.

2. Miniaturisiertes, piezoelektrisches Linear-Antriebselement (22) nach Anspruch 1,
dadurch gekennzeichnet, daß
das bewegbare Element (16) ein langgestreckter Strang ist, der zumindest in seinem unmittelbar mit dem Antriebselement (22) zusammenwirkenden Längenabschnitt flach ausgebildet ist.

3. Miniaturisiertes, piezoelektrisches Linear-Antriebselement (22) nach einem der vorstehenden Ansprüche,
dadurch gekennzeichnet, daß
der Klemmeingriffskörper (51) als fingerartiger Vorsprung an dem Klemmelement (48) ausgebildet ist und eine Schrägfläche (54) aufweist, auf die der Klemmelementbetätiger (44) einwirkt, um die Aktivierungsbewegung des Klemmelementbetätigers (44) in die Schwenkbewegung des Klemmeingriffskörpers (51) zu konvertieren.

4. Miniaturisiertes, piezoelektrisches Linear-Antriebselement (22) nach einem der vorstehenden Ansprüche,
dadurch gekennzeichnet, daß
das Klemmelement (48) ringförmig ausgebildet ist und das bewegbare Element (16) umgibt.

5. Miniaturisiertes, piezoelektrisches Linear-Antriebselement (22) nach einem der vorstehenden Ansprüche,
dadurch gekennzeichnet, daß
der Klemmeingriffskörper (51) relativ zu dem bewegbaren Element (16) eine derartige Schrägstellung (Winkel 60) besitzt, daß sich bei Kraftübertragung zwischen dem Klemmelement (48) und dem bewegbaren Element (16) eine Selbstverstärkung des Klemmeingriffs ergibt.

6. Miniaturisiertes, piezoelektrisches Linear-Antriebselement (22) nach einem der vorstehenden Ansprüche,
dadurch gekennzeichnet, daß
der Klemmeingriffskörper (51) eine Klemmfläche (56) hat, die zu dem bewegbaren Element (16) hin ausgerichtet ist.

7. Endoskop mit einem Endoskopschaft (2), der im vorderen Endbereich einen beweglichen Abschnitt (4) aufweist, wobei im Endoskopschaft (2) mindestens ein miniaturisierter Antrieb zum Bewegen des beweglichen Abschnitts (4) über ein Bewegungsübertragungselement (16) vorgesehen ist,
dadurch kennzeichnet, daß
der miniaturisierte Antrieb als ein piezoelektrisches Linear-Antriebselement (22) mit den Merkmalen gemäß einem der Ansprüche 1 bis 6 ausgebildet ist.

8. Endoskop nach Anspruch 7, gekennzeichnet durch
eine Positionsbestimmungseinrichtung (118), mittels der die Position des Bewegungsübertragungselements (16) hinsichtlich seiner Längsbewegung relativ zu dem Antriebselement (22) erfaßbar ist.

9. Endoskop nach Anspruch 8, dadurch gekennzeichnet, daß
die Positionsbestimmungseinrichtung (118) induktiv arbeitet.

10. Endoskop nach Anspruch 9, dadurch gekennzeichnet, daß
die Positionsbestimmungseinrichtung (118) zwei Spulen (118) hat, die beidseits des Antriebselements (22) angeordnet sind.

## Claims

1. Miniaturised piezo-electric linear drive element (22) for driving a movable element (16), for example for transfer of movement to the shaft of an endoscope, comprising a first and a second clamping device (38, 40) which is alternately engaged with the movable element (16) and a piezo-electric linear mover (36) arranged between the clamping devices (38, 40), **characterised in that** each clamping device (38, 40) comprises a clamping element (48), which is activated by a piezo-electric clamping element operator (44) and which has at least one clamping engagement element (51) at a slant relative to the movable element (16) and which is pivotal in the direction toward the movable element (16) under the effect of the clamping element operator (44) in order to be engaged by way of clamping with the movable element (16).

2. Miniaturised piezo-electric linear drive element (22) according to Claim 1, **characterised in that** the movable element (16) is a longitudinally stretched extrusion which is of flat design, at least in its longitudinal section which co-acts directly with the drive element (22).

3. Miniaturised piezo-electric linear drive element (22) according to one of the above claims, **characterised in that** the clamping engagement element (51) is designed as a fingerlike protrusion on the clamping element (48) comprising a slanted surface (54) onto which acts the clamping element operator (44) in order to convert the activating movement of the clamping engagement element (44) into the pivotal movement of the clamping engagement element (51).

4. Miniaturised piezo-electric linear drive element (22) according to one of the above claims, **characterised in that** the clamping element (48) is of annular design and encases the movable element (16).

5. Miniaturised piezo-electric linear drive element (22) according to one of the above claims, **characterised in that** the slanting position (angle 60) of the clamping engagement element (51) relative to the movable element (16) is such that self-reinforcement of the clamping engagement results during load transfer between the clamping element (48) and the movable element (16).

6. Miniaturised piezo-electric linear drive element (22) according to one of the above claims, **characterised in that** the clamping engagement element (51) has a clamping surface (56) oriented towards the movable element (16).

7. Endoscope with an endoscope shaft (2) which has in the front end area a movable section (4), and in the endoscope shaft (2) is provided at least one miniaturised drive for moving the movable section (4) via a movement transfer element (16), **characterised in that** the miniaturised drive is designed as a piezo-electric linear drive element (22) with the features according to one of Claims 1 to 6.

8. Endoscope according to Claim 7, **characterised by** a position detection device (118) by means of which the position of the movement transfer element (16) relative to its longitudinal movement relative to the drive element (22) can be determined.

9. Endoscope according to Claim 8, **characterised in that** the position detection device (118) operates inductively.

10. Endoscope according to Claim 9, **characterised in that** the position detection device (118) has two coils (118) arranged on both sides of the drive element (22).

## Revendications

1. Elément d'entraînement linéaire (22) piézo-électrique, miniaturisé, pour l'entraînement d'un élément déplaçable(16) par exemple pour la transmission du mouvement à une tige d'endoscope comportant
un premier et un deuxième dispositif de serrage (38, 40) qui peuvent être amenés alternativement en prise avec l'élément déplaçable (16)
et un déplaceur linéaire piézo-électrique (36) disposé entre les dispositifs de serrage (38, 40)
caractérisé en ce que
chaque dispositif de serrage (38, 40) comporte un élément de serrage (48) qui peut être activé par un actionneur (44) piézo-électrique ainsi qu'au moins un corps d'engagement par serrage (51) placé obliquement par rapport à l'élément déplaçable (16) et qui peut pivoter dans la direction de l'élément déplaçable (16), sous l'action de l'actionneur (44), pour venir en engagement par serrage avec l'élément déplaçable (16).

2. Elément d'entraînement linéaire (22) piézo-électrique miniaturisé selon la revendication 1,
caractérisé en ce que
l'élément déplaçable (16) est une barre allongée qui est plate au moins dans la partie de sa longueur qui coopère directement avec l'élément d'entraînement (22).

3. Elément d'entraînement linéaire (22) piézo-électrique miniaturisé selon l'une des revendications précédentes,
caractérisé en ce que
le corps d'engagement par serrage (51) est réalisé sous la forme d'une saillie du type doigt sur l'élément de serrage (48) et présente une surface oblique (54) sur laquelle agit l'actionneur (44) de l'élément de serrage afin de convertir le mouvement d'activation de l'actionneur (44) en mouvement de pivotement du corps d'engagement par serrage (51).

4. Elément d'entraînement linéaire (22) piézo-électrique miniaturisé selon l'une des revendications précédentes,
caractérisé en ce que
l'élément de serrage (48) est de forme annulaire et entoure l'élément déplaçable (16).

5. Elément d'entraînement linéaire (22) piézo-électrique miniaturisé selon l'une des revendications précédentes,
caractérisé en ce que
le corps d'engagement par serrage (51) présente une position oblique (angle 60) par rapport à l'élément déplaçable (16) telle qu'il se produit un autorenforcement de l'engagement par serrage en cas de transmission de forces entre l'élément de serrage (48) et l'élément déplaçable (16).

6. Elément d'entraînement linéaire (22) piézo-électrique miniaturisé selon l'une des revendications précédentes,
caractérisé en ce que
le corps d'engagement par serrage (51) présente une surface de serrage (56) qui est orientée vers l'élément déplaçable (16).

7. Endoscope avec une tige (2) qui présente un segment mobile (4) dans la zone terminale avant, un entraînement miniaturisé au moins étant prévu dans la tige (2) de l'endoscope pour le déplacement du segment mobile (4) par l'intermédiaire d'un élément de transmission de mouvement (216),
caractérisé en ce que
l'entraînement miniaturisé est réalisé sous la forme d'un élément d'entraînement linéaire (22) piézo-électrique présentant les caractéristiques selon l'une des revendications 1 à 6.

8. Endoscope selon la revendication 7, caractérisé par un dispositif de détermination de position (118) au moyen duquel la position de l'élément de transmission de mouvement (16) peut être enregistrée en ce qui concerne son mouvement longitudinal par rapport à l'élément d'entraînement (22).

9. Endoscope selon la revendication 8, caractérisé en ce que le dispositif de détermination de position (118) fonctionne de manière inductive.

10. Endoscope selon la revendication 9, caractérisé en ce que le dispositif de détermination de position (118) comporte deux bobines (118) qui sont disposées de part et d'autre de l'élément d'entraînement (22).
